# EUROPÄISCHE PATENTSCHRIFT

(11) **EP 1 086 656 B1**
(45) Veröffentlichungstag und Bekanntmachung des Hinweises auf die Patenterteilung: **09.04.2003**
(21) Anmeldenummer: 00119639.3
(22) Anmeldetag: 08.09.2000
(51) Int. Cl.: A61B 17/68

(54) **Chirurgisches Cerclageband**
Surgical cerclage strap
Bande pour cerclage chirurgical

(30) Priorität: 24.09.1999 DE 29916884 U
(43) Veröffentlichungstag der Anmeldung: 28.03.2001
(73) Patentinhaber: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 597 258
- EP-A- 0 780 096
- FR-A- 2 677 875

## Beschreibung

Um das Aufsplittern eines Knochens bei dessen chirurgischer Bearbeitung zu vermeiden oder um einen aufgesplitterten Knochen zusammenzuhalten, versieht man ihn mit einer sogenannten Cerclage. Das ist eine Umschnürung, für die man üblicherweise Draht verwendet.

Bekannt sind Cerclagebänder (WO 9426192), bei denen ein Spannschloß, das mit einem Ende eines Bandteils verbunden ist, zwei Seitenwangen aufweist, die einander gegenüberliegende Öffnungen enthalten, in denen eine Spannwelle gelagert ist, die zum Spannen des freien Bandendes bestimmt ist.

Der Erfindung liegt die Aufgabe zugrunde, ein derartiges Cerclageband so weiter zu bilden, daß es leichter handhabbar und sicherer ist.

Die erfindungsgemäße Lösung besteht in den Merkmalen des Anspruchs 1 und vorzugsweise denjenigen der Unteransprüche.

Zwar sind gewöhnliche Schlauchschellen bekannt, bei denen die Öffnungen zur Lagerung der Spannwelle einen Rastzahn enthalten und die Spannwelle im Bereich beider Öffnungen eine damit zusammenwirkende Zahnung enthält. Ein zapfenförmiger Handhabungsteil ragt aus dem Spannschloß einseitig für den Angriff eines Schlüssels heraus. Insoweit Gemeinsamkeiten der Erfindung mit derartigen Schlauchschellen bestehen, besteht der Erfindungsgedanke auf der Übertragung auf das vorliegende Anwendungsgebiet.

Das erfindungsgemäße chirurgische Cerclageband unterscheidet sich vom Stand der Technik dadurch, daß die Wickelwelle nur im Bereich einer der beiden Öffnungen eine Zahnung aufweist; der mit der anderen Öffnung zusammenwirkende Wellenbereich ist ungezahnt. Diese einseitige Anordnung der Zahnung hat den Vorteil, daß das Band verhältnismäßig leicht durch Zurückdrehen der Wickelwelle wieder gelöst werden kann. Voraussetzung für dieses Zurückdrehen ist die Lösung der Wellenzahnung von dem Rastzahn der Öffnung. Dies gelingt dadurch, daß die Wikkelwelle an der betreffenden Seite von dem Rastzahn zurückgezogen wird. Es wäre im kleinen und unübersichtlichen Operationsfeld nicht einfach, auch das andere Ende der Welle zurückzuziehen, wie es in anderen technischen Bereichen, beispielsweise bei Schlauchbindern, ohne weiteres möglich wäre.

Dem Spannband ist zweckmäßigerweise ein Umführungsinstrument beigegeben, das einen U-förmigen, steifen Teil aufweist, der um den zu bindenden Teil des Knochens geführt wird und eine Führung enthält, die es erlaubt, den Bandteil des Cerclagebands leichter um den Knochen zu führen.

Die Erfindung wird im folgenden näher unter Bezugnahme auf die Zeichnung erläutert, die ein vorteilhaftes Ausführungsbeispiel veranschaulicht. Darin zeigen:
- Fig. 1: Die Umführung des Cerclagebands um einen Knochen mittels des in
- Fig. 2: gezeigten Werkzeuges,
- Fig. 3: das um den Knochen geschlungene, noch nicht gespannte Cerclageband,
- Fig. 4: das gespannte Cerclageband,
- Fig. 5 u.6: eine Draufsicht und eine Seitenansicht des Spannschlosses in größerem Maßstab und

- Fig. 7: das Bandschloß bei herausgenommener Spannwelle.

Das Cerclageband besteht aus dem Bandteil 1 und dem allgemein mit der Ziffer 2 bezeichneten Spannschloß, das an einem Ende 3 mit einem Ende des Bandteils 1 verbunden ist. Das Spannband besteht aus Metall oder einem zugfesten Kunststoff und besitzt ausreichende Festigkeit, um die erwünschte Ringspannung auf den Knochen ausüben zu können, und hinreichende Flexibilität, um im Spannschloß aufgewickelt oder mindestens gebogen werden zu können. Für die Verbindung des Bandteils 1 mit dem Spannschloß 2 genügt es bei einem Metallband, das Ende 4 des Spannbands durch einen Schlitz des Bandschlosses zu führen und auf der Unterseite des Spannbands auf dieses zurück zu biegen.

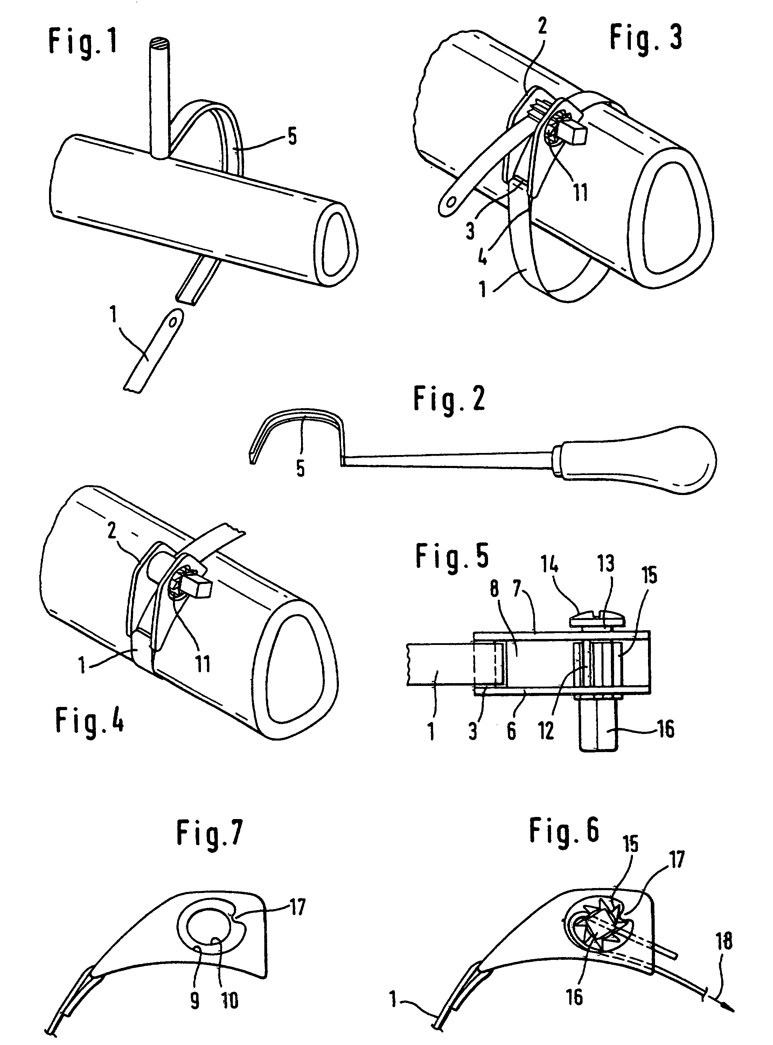

Bei seiner Anwendung wird der Bandteil 1 um den Knochen geführt. Dabei bedient man sich zweckmäßigerweise des in Fig. 1 und 2 dargestellten Werkzeuges, das eine U-förmige, im wesentliche starre Führung 5 bildet, durch die der Bandteil um den Knochen geführt werden kann.

Das Bandschloß ist ein im wesentlichen U-förmig gebogenes Blechstück mit zwei Seitenwangen 6, 7 beiderseits eines sie verbindenden Bodenteils 8. Der lichte Abstand zwischen den seitlichen Wangen 6, 7 ist wenig größer als die Breite des Bandteils 1.

Die eine Seitenwange 6 enthält eine erste Öffnung 9, die zweite Seitenwange 7 eine etwas kleinere, mit der Öffnung 9 im wesentlichen fluchtende Öffnung 10. Beide Öffnungen sind länglich, wobei die Längsrichtung etwa mit der Spannrichtung übereinstimmt. Sie sind zur Aufnahme einer Wickelwelle 11 bestimmt, die einen Schlitz 12 enthält, durch den das freie Ende des Bandteils 1 gesteckt wird, bevor es gespannt wird. Die Wickelwelle besitzt einen im wesentlichen zylindrischen Abschnitt 13, der in der zweiten Öffnung 10 liegt und darin durch einen Kopf 14 gesichert ist, der beispielsweise von einer Schraube gebildet sein kann. An den Abschnitt 13 schließt sich eine Zahnung 15 an, die im wesentlichen die gesamte zwischen den Seitenwangen 6, 7 liegende Länge der Wickelwelle 11 sowie den Bereich einnimmt, der in der ersten Öffnung 9 der Seitenwange 6 liegt. Außerhalb des Bandschloßgehäuses 6, 7, 8 erstreckt sich der zapfenförmige Handhabungsabschnitt 16 der Wickelwelle, der als Vierkant ausgebildet ist, um mittels eines nicht gezeigten Schlüssels betätigt werden zu können.

Die erste Öffnung 9 in der Seitenwange 6, in der die Zahnung 15 liegt, enthält einen Rastzahn 17 an demjenigen Ende der Öffnung 9, das dem das freie Ende des Bandteils 1 aufnehmenden Ende des Bandschlosses 2 zugewendet ist. Wie man in Fig. 6 sieht, wird die Zahnung der Wickelwelle durch den in Pfeilrichtung wirkenden Bandzug in Eingriff mit diesem Rastzahn gehalten. Dadurch wird die Wickelwelle daran gehindert, sich unter dem Bandzug zurückzudrehen. Jedoch können die Zähne sich aufgrund ihrer Sägezahnform bei der Spanndrehung der Wickelwelle an dem Rastzahn 17 vorbei bewegen, wobei die Wikkelwelle in Fig. 6 nach links ausweicht.

Nachdem der Bandteil 1 um den Knochen geführt ist, wird sein freies Ende durch den Schlitz 12 der Wickelwelle gesteckt (Fig. 3) und mit einer Zange um den Knochen straff gezogen. Danach beginnt man mit einem Schlüssel, die Wickelwelle im Uhrzeigersinn (Fig. 6) zu drehen, bis die erforderliche Bandspannung erreicht ist. Zuvor oder anschließende kann ein gegebenenfalls störendes, überstehendes Ende des Bandteils 1 abgeschnitten werden.

Will man das Cerclageband wieder entfernen, ohne es durchzuschneiden, löst man den Bandteil 1 aus dem Spannschloß 2. Zu diesem Zweck muß die Wickelwelle 11 zurückgedreht werden. Dies gelingt dadurch, daß man während des Zurückdrehens die Welle vom Rastzahn 17 entgegen der Pfeilrichtung 18 zurückzieht. Die längliche Form der Öffnungen 9, 10 erlaubt diese Bewegung der Wickelwelle. Das Zurücksetzen der Wickelwelle ist deshalb möglich, weil der Handhabungsabschnitt 16 der Öffnung 9, die den Rastzahn 17 und den gezahnten Wellenabschnitt 15 enthält, unmittelbar benachbart ist. Gegebenenfalls genügt auch eine Schwenkung der Wickelwelle um ihren Abschnitt 13; die Öffnung 10 braucht daher in manchen Fällen nicht länglich ausgebildet zu sein. In jedem Fall setzt man am Handhabungsabschnitt 16 ein Werkzeug an, mit dem man die Welle 11 vom Rastzahn 17 zurückzieht und zurückdreht, bis man das freie Ende es Bandteils 1 aus dem Spannschloß herausziehen kann.

## Patentansprüche

1. Chirurgisches Cerclageband, bestehend aus einem Bandteil (1) und einem damit an einem Ende verbundenen Spannschloß (2), das zwei Seitenwangen (6, 7) aufweist, die einen Abstand zur Aufnahme des freien Endes des Bandteils (1) einschließen und einander gegenüberliegende Öffnungen (9, 10) enthalten, in denen eine Spannwelle (11) zum Spannen des freien Endes des Bandteils (1) gelagert ist, **dadurch gekennzeichnet, daß** die Spannwelle im Bereich einer ersten (9) der beiden Öffnungen (9, 10), die einen Rastzahn (17) aufweist, eine mit dem Rastzahn (17) zusammenwirkende Zahnung (15) aufweist, während sie im Bereich (13) der zweiten (10) der beiden Öffnungen (9, 10) ungezahnt ist, daß die erste Öffnung länglich derart ausgebildet ist, daß die Spannwelle außer Eingriff ihrer Zahnung mit dem Rastzahn bewegbar ist, und daß die Welle (11) einen Handhabungsteil (16) aufweist.

2. Cerclageband nach Anspruch 1, **dadurch gekennzeichnet, daß** das Handhabungsteil von einem freistehenden Handhabungsabschnitt (16) gebildet wird.

3. Cerclageband nach Anspruch 1 oder 2, **dadurch gekennzeichnet, daß** die zweite Öffnung (10) ebenfalls länglich ist.

4. Cerclageband nach einem der Ansprüche 1 bis 3, **dadurch gekennzeichnet, daß** der Handhabungsabschnitt (16) unrund und zapfenförmig ist und auf der Seite der ersten Öffnung (9) angeordnet ist.

5. Cerclageband nach einem der Ansprüche 1 bis 4, **dadurch gekennzeichnet, daß** ihm ein Umfürungsinstrument mit einer U-förmigen Führung (5) für das Führen des Bandteils (1) um einen Knochen beigegeben ist.

## Claims

1. Surgical cerclage band, consisting of a band part (1) and of a tightening buckle (2) connected to it at one end, which tightening buckle (2) has two side walls (6, 7) which enclose a space for receiving the free end of the band part (1) and comprise openings (9, 10) which lie opposite one another and in which a tightening shaft (11) is mounted for tightening the free end of the band part (1), **characterized in that**, in the area of a first (9) of the two openings (9, 10) which has a locking tooth (17), the tightening shaft has a toothing (15) interacting with the locking tooth (17), while it is untoothed in the area (13) of the second (10) of the two openings (9, 10), **in that** the first opening is oblong so that the tightening shaft can be moved out of engagement of its toothing with the locking tooth, and **in that** the shaft (11) has a handling part (16).

2. Cerclage band according to Claim 1, **characterized in that** the handling part is formed by a free-standing handling section (16).

3. Cerclage band according to Claim 1 or 2, **characterized in that** the second opening (10) is likewise oblong.

4. Cerclage band according to one of Claims 1 to 3, **characterized in that** the handling section (16) is noncircular but peg-shaped and is arranged on the side of the first opening (9).

5. Cerclage band according to one of Claims 1 to 4, **characterized in that** it is assigned a guiding instrument with a U-shaped guide (5) for guiding the band part (1) round a bone.

## Revendications

1. Bande pour cerclage chirurgical, constituée d'une partie de bande (1) et d'un tendeur (2) qui est relié à celle-ci à une extrémité et qui comporte deux joues latérales (6, 7), lesquelles laissent une distance destinée à recevoir l'extrémité libre de la partie de bande (1) et contiennent des ouvertures (9, 10) opposées l'une à l'autre, dans lesquelles est monté un arbre de tension (11) pour tendre l'extrémité libre de la partie de bande (1), **caractérisée en ce que** l'arbre de tension comporte, dans la zone d'une première (9) des deux ouvertures (9, 10), qui présente une dent d'accrochage (17), une denture (15) coopérant avec la dent d'accrochage (17), tandis que dans la zone (13) de la deuxième (10) des deux ouvertures (9, 10), il n'est pas denté, **en ce que** la première ouverture est de forme oblongue de manière que l'arbre de tension puisse être déplacé lorsque sa denture est désengagée de la dent d'accrochage, et **en ce que** l'arbre (11) comporte une partie de manipulation (16).

2. Bande pour cerclage selon la revendication 1, **caractérisée en ce que** la partie de manipulation est formée par un tronçon de manipulation (16) libre.

3. Bande pour cerclage selon la revendication 1 ou 2, **caractérisée en ce que** la deuxième ouverture (10) est également oblongue.

4. Bande pour cerclage selon l'une des revendications 1 à 3, **caractérisée en ce que** le tronçon de manipulation (16) n'est pas circulaire et a la forme d'un tenon et est disposé sur le côté de la première ouverture (9).

5. Bande pour cerclage selon l'une des revendications 1 à 4, **caractérisé en ce qu'**il lui est adjoint un instrument d'encerclement avec un guide (5) en U pour guider la partie (1) de la bande autour d'un os.
